# EUROPEAN PATENT APPLICATION

(11) **EP 4 563 994 A1**
(43) Date of publication of application: **04.06.2025**
(21) Application number: 23846368.1
(22) Date of filing: 20.07.2023
(51) Int. Cl.: G01N 27/414, G01N 27/416

(54) **DEVICE, HYDROXIDE ION CONCENTRATION MEASUREMENT APPARATUS COMPRISING SAID DEVICE, AND METHOD FOR MEASURING HYDROXIDE ION CONCENTRATION USING SAID DEVICE**

(30) Priority: 29.07.2022 JP 2022121592
(71) Applicant: Mitsui Mining & Smelting Co., Ltd., Shinagawa-ku Tokyo 141-8584 (JP)
(72) Inventor: TOKUCHI Shigeki, Ageo-shi, Saitama 362-0021 (JP)
(74) Representative: Dehns
(86) International application number: PCT/JP2023/026642
(87) International publication number: WO 2024/024637

(57) **Abstract**

Provided are an apparatus and a method for measuring a concentration of hydroxide ions which are capable of easily and quickly measuring the concentration of the hydroxide ions with high accuracy and measuring the concentration of hydroxide ions at relatively low cost. An insulating substrate, at least one insulating composite layer, and a liquid storage portion capable of holding an ion-containing liquid are provided, the insulating composite layer includes a pair of electrodes and a semiconductor layer in contact with the pair of electrodes, and a layered double hydroxide layer having an OH⁻ ion conductivity of 1 × 10⁻⁵ S/cm or more is provided between the insulating composite layer and the liquid storage portion.

## Description

### Technical Field

The present invention relates to a device in which the ease of flowing of a current changes in correspondence with a concentration of hydroxide ion (OH⁻), and more specifically, to a measurement apparatus and a measurement method for measuring the concentration of hydroxide ion (OH⁻) of a test subject based on such a change in conductivity.

### Background Art

A hydroxide ion (OH⁻) is one of basic and important substances in various fields including material chemistry and life science. A chemical reaction, a chemical equilibrium, and an electrochemical phenomena cannot be understood without considering the presence and chemical behavior of hydroxide ions.

As a method for measuring the concentration of such hydroxide ions, a pH titration method, a method using a pH meter, and others are conventionally known.

In addition, Patent Literature 1 discloses that hydroxide ions in a solution are separated by chromatography, and the concentration of the hydroxide ions is measured by using an electrical conductivity method or a light absorbance method.

### Citation List

### Patent Literature

Patent Literature 1: JP 2002-5916 A

### Summary of Invention

### Technical Problem

In the pH titration method, the concentration of hydroxide ions can be measured with high accuracy, but in order to accurately perform the titration, it is difficult to easily and accurately measure the concentration of hydroxide ions because it requires skill to perform the titration.

In the pH meter, the concentration of hydrogen ions can be measured by a potential difference between a glass electrode and a comparison electrode, and the concentration of hydroxide ions can be measured on the basis of the concentration of hydrogen ions, and thus the concentration of hydroxide ions can be easily and quickly measured, but the accuracy is not high.

In addition, in the method for separating hydroxide ions by the chromatograph disclosed in Patent Literature 1, although the measurement accuracy is high, it is essential to create a separation column, and it is necessary to select a separation column depending on a type of a solvent and a pH range, and there are also problems, for example, that cracking and others are likely to occur in the separation column and care is required for handling, that the chromatograph is expensive, and that measurement cannot be easily performed.

In consideration of such a current situation, an object of the present invention is to provide an apparatus and a method for measuring a concentration of hydroxide ions which are capable of easily and quickly measuring the concentration of hydroxide ions with high accuracy and measuring the concentration of hydroxide ions at a relatively low cost.

In addition, another object of the present invention is to provide a device that can be used in an apparatus and a method for measuring the concentration of hydroxide ions as described above and has a conductivity that varies depending on the concentration of hydroxide ions.

### Solution to Problem

The present invention has been made to solve the problems in the related art as described above, and the device of the present invention, the hydroxide ion measurement apparatus including the device, and the method for measuring the concentration of hydroxide ions by using the device include those configured as follows.

[1] A device including an insulating substrate; at least one insulating composite layer; and a liquid storage portion capable of holding an ion-containing liquid, wherein
   the insulating composite layer includes a pair of electrodes and a semiconductor layer in contact with the pair of electrodes, and
   a layered double hydroxide layer having an OH⁻ ion conductivity of 1 × 10⁻⁵ S/cm or more is provided between the insulating composite layer and the liquid storage portion.
[2] The device according to [1], wherein the insulating composite layer further includes a third electrode, and an insulating layer is provided between the pair of electrodes and the semiconductor layer, and the third electrode.
[3] The device according to [1] or [2], wherein field-effect mobility of the semiconductor layer is 20 cm²/Vs or more.
[4] The device according to any one of [1] to [3], wherein the semiconductor layer contains at least one of oxygen, sulfur, nitrogen, boron, and carbon.
[5] The device according to [4], wherein the semiconductor layer contains at least one of indium, zinc, tin, and a Group 13 element.
[6] The device according to any one of [1] to [5], wherein the semiconductor layer includes at least one of graphene, carbon nanotube, boron nitride, metal dichalcogenide, and phospholene.
[7] The device according to any one of [1] to [6], wherein
   the layered double hydroxide layer contains at least one of pyreneboronic acid, 1-pyrenebutyric acid N-hydroxysuccinimide ester, and
   a general formula of the layered double hydroxide layer is [M^{II}₁₋ₓM'^{III}ₓ(OH)₂]^{x+} where M^{II} is selected from the group consisting of Ca²⁺, Mg²⁺, Mn²⁺, Fe²⁺, Co²⁺, Ni²⁺, Cu²⁺, Zn²⁺, and 2Li⁺, and M^{III} is selected from the group consisting of Al³⁺, Mn³⁺, Fe³⁺, Co³⁺, Cr³⁺, and Ni³⁺.
[8] The device according to any one of [1] to [7], further including a protective layer between the layered double hydroxide layer and the semiconductor layer.
[9] The device according to any one of [1] to [8], wherein a thickness of the semiconductor layer is 0.5 µm or less.
[10] The device according to any one of [1] to [9], wherein a maximum height Sz of a surface of the semiconductor layer on the layered double hydroxide layer side is 0.05 µm or less.
[11] The device according to any one of [1] to [10], wherein an arithmetic mean height Sa of a surface of the semiconductor layer on the layered double hydroxide layer side is 0.03 µm or less.
[12] A hydroxide ion concentration measurement apparatus, including the device according to any one of [1] to [11].
[13] A method for measuring a concentration of hydroxide ions by using the device according to any one of [1] to [11].
[14] The method for measuring a concentration of hydroxide ions according to [13], wherein the concentration of hydroxide ions in any one of river water, sea water, purified water, washing water, and industrial waste water is measured.

### Advantageous Effects of Invention

According to the present invention, it is possible to easily and quickly measure the concentration of hydroxide ions in a solution that is a subject with high accuracy by using a device in which the conductivity varies in correspondence with the concentration of hydroxide ions (OH⁻) .

### Brief Description of Drawings

Fig. 1 is a schematic view illustrating a configuration of a hydroxide ion concentration measurement apparatus in the present embodiment.
Fig. 2 is a schematic view illustrating a configuration of a hydroxide ion concentration measurement apparatus according to another embodiment.

### Description of Embodiments

Hereinafter, embodiments (examples) of the present invention will be described in more detail with reference to the drawings.

Note that, in an apparatus and a method for measuring a concentration of hydroxide ions according to the present invention, it is possible to measure a concentration of hydroxide ions in, for example, river water, sea water, purified water, washing water, industrial waste water, and others as an ion-containing liquid which is a test subject, and for example, it is possible to constantly monitor the water quality thereof, and it is possible to contribute to universal and equal access of safe and inexpensive drinking water.

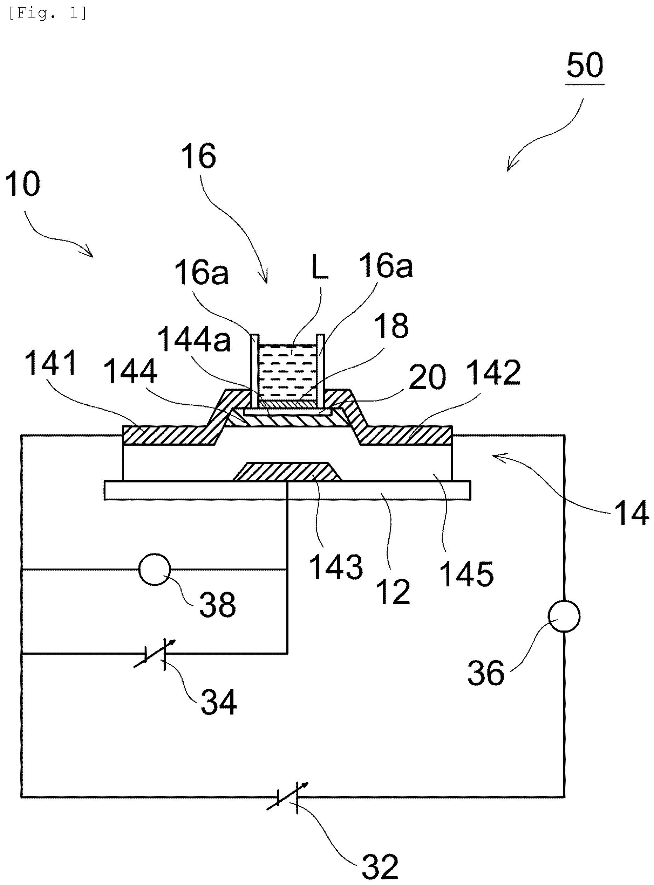

Fig. 1 is a schematic view illustrating a configuration of a hydroxide ion concentration measurement apparatus according to the present embodiment.

As shown in Fig. 1, a hydroxide ion concentration measurement apparatus 50 of the present embodiment includes a device 10 including an insulating substrate 12, at least one insulating composite layer 14, and a liquid storage portion 16 capable of holding an ion-containing liquid. Note that, the device 10 configured as described below can also be, for example, a semiconductor element such as a field effect transistor (FET) or a metal-oxide semiconductor field effect transistor (MOSFET).

The insulating substrate 12 is, for example, a substrate formed from an insulating material such as glass or ceramics.

The insulating composite layer 14 includes a pair of electrodes (a first electrode 141 and a second electrode 142) and a semiconductor layer 144 in contact with the pair of electrodes 141 and 142.

In addition, a layered double hydroxide layer 18 is provided between the insulating composite layer 14 and the liquid storage portion 16. The layered double hydroxide layer 18 is in contact with an ion-containing liquid L held in the liquid storage portion 16 and conducts hydroxide ions (OH⁻) contained in the ion-containing liquid L. The OH⁻ ion conductivity of the layered double hydroxide layer 18 in the present embodiment is 1 × 10⁻⁵ S/cm or more, more preferably 1 × 10⁻⁴ S/cm or more, still more preferably 5.0 × 10⁻⁴ S/cm or more, and most preferably 1 × 10⁻³ S/cm or more. An upper limit value of the OH⁻ ion conductivity of the layered double hydroxide layer 18 is not particularly limited, but is generally 1.0 × 10 S/cm.

In addition, a protective layer 20 is provided between the layered double hydroxide layer 18 and the semiconductor layer 144, and the layered double hydroxide layer 18 and the electrodes 141 and 142 are electrically insulated from each other by, for example, a partition portion 16a of the liquid storage portion 16.

In addition, the layered double hydroxide layer 18 preferably contains at least one of pyreneboronic acid, 1-pyrenebutyric acid N-hydroxysuccinimide ester, and
a general formula of the layered double hydroxide layer 18 is [M^{II}₁₋ₓM'^{III}ₓ(OH)₂]^{x+} where M^{II} is selected from the group consisting of Ca²⁺, Mg²⁺, Mn²⁺, Fe²⁺, Co²⁺, Ni²⁺, Cu²⁺, Zn²⁺, and 2Li⁺, and M^{III} is selected from the group consisting of Al³⁺, Mn³⁺, Fe³⁺, Co³⁺, Cr³⁺, and Ni³⁺.

In addition, the semiconductor layer 144 preferably has a field-effect mobility of 20 cm²/Vs or more, particularly 60 cm²/Vs or more.

The semiconductor layer 144 can contain at least one of oxygen, sulfur, nitrogen, boron, and carbon. In addition, the semiconductor layer 144 preferably further contains at least one of indium, zinc, tin, and a Group 13 element.

The semiconductor layer 144 can contain at least one of graphene, carbon nanotube, boron nitride, metal dichalcogenide, or phospholene.

More specifically, indium-zinc-oxide (IZO) such as Ta-IZO can be used as the semiconductor layer 144.

In addition, the smaller the thickness of the semiconductor layer 144, the larger a variation in conductivity of a surface layer, and thus, as described later, a variation in moving charges becomes larger, and measurement accuracy is improved. The thickness of the semiconductor layer 144 is preferably 0.5 µm or less, more preferably 0.1 µm or less, and particularly preferably 0.05 µm or less. A lower limit value of the thickness of the semiconductor layer 144 is not particularly specified, but is generally 0.005 µm or more.

A surface 144a of the semiconductor layer 144 on the layered double hydroxide layer 18 side is preferably as smooth as possible. When the surface 144a of the semiconductor layer 144 is not smooth, for example, a gap is generated between the surface and the protective layer 20, or the protective layer 20 is discontinuously formed, and thus adhesion between the semiconductor layer 144 and the layered double hydroxide layer 18 deteriorates, and a current flows only in a part of the surface 144a of the semiconductor layer 144. For this reason, the measurement accuracy decreases or the operation becomes unstable.

Specifically, a maximum height Sz of the surface 144a of the semiconductor layer 144 is preferably 0.05 µm or less, more preferably 0.01 µm or less, and most preferably 0.003 µm or less. A lower limit value of the maximum height Sz is not particularly specified, but is generally 0.0005 µm or more. In addition, an arithmetic mean height Sa of the surface 144a of the semiconductor layer 144 on the layered double hydroxide layer 18 side is preferably 0.03 µm or less, more preferably 0.005 µm or less, and most preferably 0.002 µm or less. The lower limit value of the arithmetic mean height Sa is not particularly specified, but is generally 0.0002 µm or more.

Here, the maximum height Sz and the arithmetic mean height Sa are parameters of surface roughness defined by ISO25178, and such parameters can be measured by, for example, a 3D surface roughness profile analyzer (NexView manufactured by Zygo Corporation). Note that, the measurement conditions in this case are preferably performed according to the following.

Measurement is performed under conditions of an objective lens of 50 times, a zoom lens of 20 times, and a measurement range of 89 µm × 87 µm in accordance with ISO25178. A roughness curve in a range of 3 µm × 3 µm is extracted from an obtained three-dimensional surface shape, the roughness curve is corrected under the following correction conditions by an analysis program "Mx" attached to a 3D surface roughness shape measuring device, and the maximum height Sz and the arithmetic mean height Sa are calculated.

### <Correction Conditions>

- Remove: Form Remove
- Filter Type: Spline
- Filter: Low Pass
- Type: Gaussian Spline Auto

In addition, the protective layer 20 is provided to protect the semiconductor layer 144 from the ion-containing liquid L. By providing the protective layer 20, the semiconductor layer 144 can be prevented from, for example, being corroded, and the durability and reliability of the semiconductor layer 144 can be improved. As a material of the protective layer 20, a known material can be applied as long as the material has insulating properties, and a material having corrosion resistance is preferable. For example, Ta₂O₅, Si₃N₄, SiO₂, and others are used, and the thickness is preferably from 0.01 µm to 0.5 µm, and more preferably from 0.03 µm to 0.2 µm.

In addition, in the present embodiment, the insulating composite layer 14 further includes a third electrode 143. As such a configuration, by applying a voltage from the third electrode 143, the current flowing between the first electrode 141 and the second electrode 142 can be increased, and the measurement accuracy can be enhanced. In addition, the pair of electrodes 141 and 142 and the semiconductor layer 144 includes an insulating layer 145 between these and the third electrode 143. As the material of the insulating layer 145, a known material can be applied as long as the material can be electrically insulated, and for example, various ceramics and resins can be used, and the thickness is preferably from 0.01 µm to 0.5 µm, and more preferably from 0.03 µm to 0.2 µm.

In addition, the measurement apparatus 50 of the present embodiment includes a variable voltage source 32 for applying a voltage between the first electrode 141 and the second electrode 142, a variable voltage source 34 for applying a voltage between the first electrode 141 and the third electrode 143, an ammeter 36 for measuring a current value between the first electrode 141 and the second electrode 142, and a voltmeter 38 for measuring a voltage value between the first electrode 141 and the third electrode 143.

In the device 10 of the present embodiment configured as described above, the ease of flow of the current in the semiconductor layer 144 varies in correspondence with the concentration of the OH⁻ ion in the ion-containing liquid L held in the liquid storage portion 16. Specifically, as the amount of OH⁻ conducting through the layered double hydroxide layer 18 increases, a current easily flows through the semiconductor layer 144.

Therefore, in the measurement apparatus 50 of the present embodiment, a predetermined voltage, for example, a voltage of approximately 1 V to 10 V is applied between the first electrode 141 and the second electrode 142 by the variable voltage source 32 in a state in which the ion-containing liquid L that is a subject is stored in the liquid storage portion 16, a voltage to be applied between the first electrode 141 and the third electrode 143 is gradually increased by the variable voltage source 34, and a voltage value (V_{TH}) between the first electrode 141 and the third electrode 143 when a current that flows between the first electrode 141 and the second electrode 142 and detected by the ammeter 36 reaches a predetermined current value is measured by the voltmeter 38.

By creating a correspondence with the voltage value V_{TH} as a calibration curve for the ion-containing liquid L whose the concentration of OH⁻ ions is known, the concentration of OH⁻ ions of the subject that is the ion-containing liquid L whose the concentration of OH⁻ ions is unknown can be measured.

In addition, in the measurement apparatus 50 of the present embodiment, in a state in which the ion-containing liquid L that is a subject is stored in the liquid storage portion 16, a current value (I_{DS}) of a current flowing between the first electrode 141 and the second electrode 142, that is, through the semiconductor layer 144 can be measured by the ammeter 36 in a state in which a predetermined voltage, for example, a voltage of approximately 1 V is applied between the first electrode 141 and the second electrode 142 by the variable voltage source 32.

By preparing a calibration curve of the current value I_{DS} and the ion-containing liquid L whose the concentration of OH⁻ ions is known in advance, it is possible to measure the concentration of OH⁻ ions of the ion-containing liquid L whose the concentration OH⁻ ions is unknown.

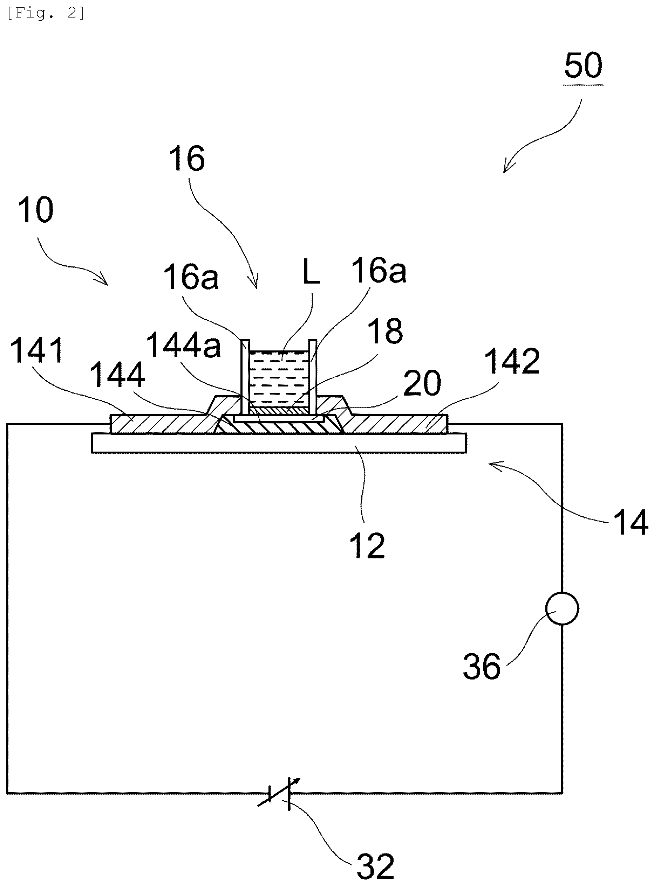

Fig. 2 is a schematic view illustrating a configuration of a hydroxide ion concentration measurement apparatus according to another embodiment.

A hydroxide ion concentration measurement apparatus 50 shown in Fig. 2 basically has the same configuration as the hydroxide ion concentration measurement apparatus 50 shown in Fig. 1, and the same components are denoted by the same reference numerals, and the detailed description thereof will be omitted.

In the hydroxide ion concentration measurement apparatus 50 of the present embodiment, the device 10 does not include the third electrode 143 and the insulating layer 145.

Even in the case of such a configuration, in a state in which the ion-containing liquid L that is a subject is stored in the liquid storage portion 16, the current value (I_{DS}) of the current flowing between the first electrode 141 and the second electrode 142, that is, through the semiconductor layer 144 is measured by the ammeter 36 in a state where a predetermined voltage smaller than the voltage value V_{TH} is applied between the first electrode 141 and the second electrode 142 by the variable voltage source 32, and the concentration of OH⁻ ions in the ion-containing liquid L that is a subject whose the concentration of OH⁻ ions is unknown can be measured by using the calibration curve of the current value I_{DS} and the ion-containing liquid L whose the concentration of OH⁻ ions is known.

Although the preferred embodiments of the present invention have been described above, the present invention is not limited thereto, and various modifications can be made without departing from the object of the present invention.

### Reference Signs List

- 10: Device
- 12: Insulating substrate
- 14: Insulating composite layer
- 141: First electrode
- 142: Second electrode
- 143: Third electrode
- 144: Semiconductor layer
- 144a: Surface
- 145: Insulating layer
- 16: Liquid storage portion
- 16a: Partition portion
- 18: Layered double hydroxide layer
- 20: Protective layer
- 32: Variable voltage source
- 34: Variable voltage source
- 36: Ammeter
- 38: Voltmeter
- 50: Measurement apparatus
- L: Ion-containing liquid

## Claims

1. A device comprising: an insulating substrate; at least one insulating composite layer; and a liquid storage portion capable of holding an ion-containing liquid, wherein
the insulating composite layer includes a pair of electrodes and a semiconductor layer in contact with the pair of electrodes, and
a layered double hydroxide layer having an OH⁻ ion conductivity of 1 × 10⁻⁵ S/cm or more is provided between the insulating composite layer and the liquid storage portion.

2. The device according to claim 1, wherein
the insulating composite layer further includes a third electrode, and
an insulating layer is provided between the pair of electrodes and the semiconductor layer, and the third electrode.

3. The device according to claim 1, wherein field-effect mobility of the semiconductor layer is 20 cm²/Vs or more.

4. The device according to claim 1, wherein the semiconductor layer contains at least one of oxygen, sulfur, nitrogen, boron, and carbon.

5. The device of claim 4, wherein the semiconductor layer contains at least one of indium, zinc, tin, and a Group 13 element.

6. The device of claim 4, wherein the semiconductor layer includes at least one of graphene, carbon nanotube, boron nitride, metal dichalcogenide, and phospholene.

7. The device according to claim 1, wherein
the layered double hydroxide layer contains at least one of pyreneboronic acid, 1-pyrenebutyric acid N-hydroxysuccinimide ester, and
a general formula of the layered double hydroxide layer is [M^{II}₁₋ₓM'^{III}ₓ(OH)₂]^{x+} where M^{II} is selected from the group consisting of Ca²⁺, Mg²⁺, Mn²⁺, Fe²⁺, Co²⁺, Ni²⁺, Cu²⁺, Zn²⁺, and 2Li⁺, and M'^{III} is selected from the group consisting of Al³⁺, Mn³⁺, Fe³⁺, Co³⁺, Cr³⁺, and Ni³⁺.

8. The device of claim 1, further comprising a protective layer between the layered double hydroxide layer and the semiconductor layer.

9. The device according to claim 1, wherein a thickness of the semiconductor layer is 0.5 µm or less.

10. The device according to claim 1, wherein a maximum height Sz of a surface of the semiconductor layer on the layered double hydroxide layer side is 0.05 µm or less.

11. The device according to claim 1, wherein an arithmetic mean height Sa of a surface of the semiconductor layer on the layered double hydroxide layer side is 0.03 µm or less.

12. A hydroxide ion concentration measurement apparatus, comprising the device according to any one of claims 1 to 11.

13. A method for measuring a concentration of hydroxide ions by using the device according to any one of claims 1 to 11.

14. The method for measuring a concentration of hydroxide ions according to claim 13, wherein the concentration of hydroxide ions in any one of river water, sea water, purified water, washing water, and industrial waste water is measured.
